# EUROPÄISCHE PATENTSCHRIFT

(11) **EP 1 773 125 B1**
(45) Veröffentlichungstag und Bekanntmachung des Hinweises auf die Patenterteilung: **21.05.2008**
(21) Anmeldenummer: 05766973.1
(22) Anmeldetag: 19.07.2005
(51) Int. Cl.: A01N 43/713, C07D 257/04

(54) **5-IODTETRAZOLE**
5-IODOTETRAZOLES
5-IODE TETRAZOLE

(30) Priorität: 30.07.2004 DE 102004037366
(43) Veröffentlichungstag der Anmeldung: 18.04.2007
(73) Patentinhaber: LANXESS Deutschland GmbH, 51369 Leverkusen (DE)
(72) Erfinder: UHR, Hermann, 51373 Leverkusen (DE); BRUNS, Rainer, 51373 Leverkusen (DE); VOGL, Erasmus, 51373 Leverkusen (DE); KUGLER, Martin, 42799 Leichlingen (DE); KRETSCHIK, Oliver, 51063 Leverkusen (DE); NEUMANN, Bernhard, 06110 Halle/S. (DE)
(86) Internationale Anmeldenummer: PCT/EP2005/007824
(87) Internationale Veröffentlichungsnummer: WO 2006/012996

(56) Entgegenhaltungen:
- EP-A- 0 107 859
- DATABASE CHEMABS [Online] CHEMICAL ABSTRACTS SERVICE, COLUMBUS, OHIO, US; Y.SATOH ET AL.: "Application of 5-lithiotetrazoles in organic synthesis" XP002351051 gefunden im STN-INTERNATIONAL Database accession no. 123:55771 & TETRAHEDRON LETTERS, Bd. 36, Nr. 11, 1995, Seiten 1759-1762,
- DATABASE CHEMABS [Online] CHEMICAL ABSTRACTS SERVICE, COLUMBUS, OHIO, US; Y.V. GRIGOR'EV ET AL.: "Synthesis of 5-iodotetrazoles" XP002351052 gefunden im STN-INTERNATIONAL Database accession no. 116:255555 & VESTSI AKADEMII NAVUK BELARSI, SERYYA KHIMICHNYKH NAVUK, Nr. 1, 1992, Seiten 73-77,
- DATABASE CHEMABS [Online] CHEMICAL ABSTRACTS SERVICE, COLUMBUS, OHIO, US; P.N.GAPONIK ET AL.: "Direct iodination of 1-alkyltetrazoles - a simple route to 1-alkyl-5-iodotetrazoles" XP002351053 gefunden im STN-INTERNATIONAL Database accession no. 111:194674 & KHIMIYA GETEROTSIKLICHESKIKH SOEDINENII, Nr. 12, 1988, Seite 1699,
- DATABASE CHEMABS [Online] CHEMICAL ABSTRACTS SERVICE, COLUMBUS, OHIO, US; V.V.NEDEL'KO ET AL.: "Thermal decomposition of C-iodotetrazoles" XP002351054 gefunden im STN-INTERNATIONAL Database accession no. 125:10018 & IZVESTIYA AKADEMII NAUK, SERIYA KHIMICHESKAYA, Nr. 1, 1996, Seiten 68-71,
- DATABASE CHEMABS [Online] CHEMICAL ABSTRACTS SERVICE, COLUMBUS, OHIO, US; C.R.JACOBSON ET AL.: "Tetrazole chemistry. II. The synthesis and reactions of 1-phenyl-5-acetyltetrazole" XP002351055 gefunden im STN-INTERNATIONAL Database accession no. 49:84232 & JOURNAL OF ORGANIC CHEMISTRY, Bd. 19, 1954, Seiten 1652-1661,
- DATABASE CHEMABS [Online] CHEMICAL ABSTRACTS SERVICE, COLUMBUS, OHIO, US; V.V.NEDLE'KO ET AL.: "Thermal decomposition of 1-ethyl-5-iodotetrazole" XP002351056 gefunden im STN-INTERNATIONAL Database accession no. 122:264790 & IZVESTIYA AKADEMII NAUK, SERIYA KHIMICHESKAYA, Nr. 11, 1994, Seiten 1923-1926,

## Beschreibung

Die Erfindung betrifft die Verwendung neuer und bekannter 5-Iod-tetrazole als Biozide zum Schutz von Pflanzen und technischen Materialien, neue 5-Iod-tetrazole sowie Verfahren zu ihrer Herstellung.

Aus der Literatur sind bereits einige Iodtetrazole und Wege zu deren Herstellung bekannt. Eine biologische Wirkung ist in keinem der vorbeschriebenen Fälle erwähnt.

Die Herstellung von 5-Iod-1-phenyl-tetrazol aus 5-Acetyl-1-phenyl-tetrazol und Natriumhypoiodit ist bekannt (vgl. C. A. Jacobson et al., J. Org. Chem. 1954, 19, 1652).

Ein weiterer Herstellweg von 5-Iod-1-phenyl-tetrazol aus Phenylisonitril und IN₃ ist beschrieben (vgl. F. W. Fowler et al.; J. Am. Chem. Soc. 1967, 89, 2077 ; W. L. Collibee et al., J. Org. Chem. 1995, 60, 468).

5-Iod-tetrazole lassen sich auch erhalten, wenn man 1-substituierte 5-Tetrazolyllithium Verbindungen bei niedrigen Temperaturen mit Iod umsetzt (vgl. R. Raap, Can. J. Chem. 1971, 49, 2139; Satoh, Yoshitaka; Tetrahedron Lett., 1995, 36, 1759; Satoh, Yoshitaka, Synlett 1998, 528).

Ein weiteres Verfahren zur Herstellung von 1-Alkyl-5-iod-tetrazolen geht von 1-Alkyltetrazolen aus, die in Eisessig, welcher KMnO₄ und H₂SO₄ enthält, mit Iod behandelt werden (vgl. P. N. Gaponik, Khimiya Geterotsiklicheskikh Soedinenii 1988, 1699).

In der Datenbank-Zusammenfassung CHEMABS CA116:255555 & Y.V. GRIGOR'EV ET AL.: "Synthesis of 5-iodotetrazoles", Vestsi Akademii Navuk Belarsi, Seryya Khimichnykh Navuk, 1, 1992, 73-77, wird 5,5'-Bisiod-1,1'-(ethandiyl)-1*H*-tetrazol beschrieben.

EP 0 107 859 A **beschreibt Triiodalkyl- oder Triodpropargyl-substituierte Tetrazole, sowie antibakterielle und antifungizide Zubereitungen hiermit zur Verwendung im medizinischen, landwirtschaftlichen und industriellen Bereich.**

Es wurde nun gefunden, dass bestimmte, in 1-Stellung substituierte, 5-Iod-tetrazole sehr gut als Mikrobizide zum Schutz von Pflanzen und Materialien geeignet sind.

Gegenstand der vorliegenden Erfindung ist daher die Verwendung von Verbindungen der Formel (I)

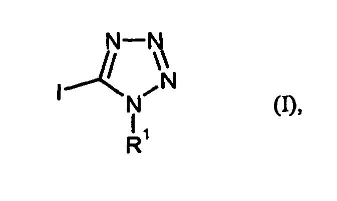

Bevorzugt ist die Verwendung von Verbindungen der allgemeinen Formel (I), in welcher
- R¹: für Wasserstoff, für geradkettiges oder verzweigtes, unsubstituiertes oder ein- oder mehrfach gleich oder verschieden substituiertes C₁-C₈-Alkyl, für geradkettiges oder verzweigtes, unsubstituiertes oder ein- oder mehrfach gleich oder verschieden substituiertes C₂-C₈-Alkenyl oder für geradkettiges oder verzweigtes, unsubstituiertes oder ein-oder mehrfach gleich oder verschieden substituiertes C₂-C₈-Alkinyl steht,
wobei die Substituenten für die ein- oder mehrfach gleich oder verschieden substituierten Alkyl-, Alkenyl- und Alkinylreste ausgewählt sind aus der Reihe Halogen; Nitro; Cyano; Hydroxy; unsubstituiertes C₁-C₆-Alkoxy; C₁-C₆-Alkoxy welches 1- bis 9-fach, gleich oder verschieden durch Halogen substituiert ist; unsubstituiertes C₁-C₆-Alkylthio; C₁-C₆-Alkylthio welches 1- bis 9-fach, gleich oder verschiedenen durch Halogen substituiert ist; Amino; Monoalkylamino mit geradkettigen oder verzweigten C₁-C₆-Alkylresten; Dialkylamino mit gleichen oder verschiedenen, geradkettigen oder verzweigten C₁-C₆-Alkylresten; unsubstituiertes Phenyl; Phenyl welches ein bis 5-fach, gleich oder verschieden substituiert ist durch Halogen, Nitro, Cyano, Alkyl, Halogenalkyl, Alkoxy, Halogenalkoxy, Alkylthio, Halogenalkylthio, Acyl, Acyloxy, Alkoxy-carbonyl, Carboxyl, Amino, Monoalkylamino oder Dialkylamino;
oder
- R¹: für unsubstituiertes Phenyl oder für Phenyl steht, welches ein bis 5-fach, gleich oder verschieden substituiert ist durch Halogen, Nitro, Cyano, Hydroxy, Alkyl, Halogenalkyl, Alkoxy, Halogenalkoxy, Alkylthio, Halogenalkylthio, Acyl, Acyloxy, Alkoxycarbonyl, Carboxyl, Amino, Monoalkylamino oder Dialkylamino.

Besonders bevorzugt ist die Verwendung von Verbindungen der allgemeinen Formel (I), in welcher
- R¹: für Wasserstoff, für geradkettiges oder verzweigtes, unsubstituiertes oder ein- bis vierfach, gleich oder verschieden substituiertes C₁-C₈-Alkyl, für unsubstituiertes oder ein- bis vierfach, gleich oder verschieden substituiertes C₂-C₆-Alkenyl oder für unsubstituiertes oder ein- bis vierfach, gleich oder verschieden substituiertes C₂-C₆-Alkinyl steht,
wobei die Substituenten für die ein- bis vierfach, gleich oder verschieden substituierten C₁-C₈-Alkyl-, C₂-C₆-Alkenyl- und C₂-C₆-Alkinylreste ausgewählt sind aus der Reihe Fluor; Chlor; Brom; Nitro; Cyano; Hydroxy; unsubstituiertes C₁-C₄-Alkoxy; C₁-C₄-Alkoxy welches 1- bis 5-fach, gleich oder verschieden durch Fluor, Chlor oder Brom substituiert ist; unsubstituiertes C₁-C₄-Alkylthio; C₁-C₄-Alkylthio welches 1- bis 5-fach, gleich oder verschiedenen durch Fluor, Chlor oder Brom substituiert ist; Amino; Monoalkylamino mit geradkettigen oder verzweigten C₁-C₄-Alkylresten; Dialkylamino mit gleichen oder verschiedenen, geradkettigen oder verzweigten C₁-C₄-Alkylresten; unsubstituiertes Phenyl; Phenyl welches ein bis vierfach, gleich oder verschieden substituiert ist durch Fluor, Chlor, Brom, Nitro, Cyano, Hydroxy, C₁-C₄-Alkyl, C₁-C₄-Halogenalkyl welches 1- bis 5-fach gleich oder verschieden durch Fluor, Chlor oder Brom substituiert ist, C₁-C₄-Alkoxy, C₁-C₄-Halogenalkoxy welches 1- bis 5-fach gleich oder verschieden durch Fluor, Chlor oder Brom substituiert ist, C₁-C₄-Alkylthio, C₁-C₄-Halogenalkylthio welches 1- bis 5-fach gleich oder verschieden durch Fluor, Chlor oder Brom substituiert ist, C₁-C₆-Acyl, C₁-C₆-Acyloxy, C₁-C₆-Alkoxycarbonyl, Carboxyl, Amino, Monoalkylamino mit geradkettigen oder verzweigten C₁-C₄-Alkylresten, Dialkylamino mit gleichen oder verschiedenen, geradkettigen oder verzweigten C₁-C₄-Alkylresten,
oder
- R¹: für unsubstituiertes Phenyl oder für Phenyl steht, welches ein bis vierfach, gleich oder verschieden substituiert ist durch Fluor, Chlor, Brom, Nitro, Cyano, Hydroxy, C₁-C₄-Alkyl, C₁-C₄-Halogenalkyl welches 1- bis 5-fach gleich oder verschieden durch Fluor, Chlor oder Brom substituiert ist, C₁-C₄-Alkoxy, C₁-C₄-Halogenalkoxy welches 1- bis 5-fach gleich oder verschieden durch Fluor, Chlor oder Brom substituiert ist, C₁-C₄-Alkylthio, C₁-C₄-Halogenalkylthio welches 1- bis 5-fach gleich oder verschieden durch Fluor, Chlor oder Brom substituiert ist, C₁-C₄-Acyl, C₁-C₄-Acyloxy, C₁-C₄-Alkoxycarbonyl, Carboxyl, Amino, Monoalkylamino mit geradkettigen oder verzweigten C₁-C₄-Alkylresten, Dialkylamino mit gleichen oder verschiedenen, geradkettigen oder verzweigten C₁-C₄-Alkylresten,

Ganz besonders bevorzugt ist die Verwendung von Verbindungen der Formel (I), in welcher
- R¹: für Wasserstoff, Methyl, Ethyl, n-Propyl, iso-Propyl, n-Butyl, iso-Butyl, sec-Butyl, tert.-Butyl, n-Pentyl, n-Hexyl, n-Heptyl, n-Octyl, Allyl, Vinyl, Propargyl steht, wobei die genannten Alkylreste jeweils gegebenenfalls ein- bis vierfach, gleich oder verschieden substituiert sind durch Fluor, Chlor, Brom, Nitro, Cyano, Hydroxy, Methoxy, Ethoxy, n-Propoxy, iso-Propoxy, n-Butoxy, iso-Butoxy, sec-Butoxy, tert-Butoxy, Trifluormethoxy, Methylthio, Ethylthio, n-Propylthio, iso-Propylthio, Trifluormethylthio, Amino, Methylamino, Ethylamino, n-Propylamino, iso-Propylamino, Dimethylamino, Diethylamino, Methylethylamino, Di-n-propylamino Di-iso-propylamino, unsubstituiertes Phenyl, Phenyl welches ein- bis dreifach durch Fluor, Chlor, Brom, Nitro, Cyano, Methylthio, Ethylthio, n-Propylthio, iso-Propylthio, Trifluormethylthio, Amino, Methylamino, Ethylamino, n-Propylamino, iso-Propylamino, Dimethylamino, Diethylamino, Methylethylamino, Di-n-propylamino Di-iso-propylamino, unsubstituiertes Phenyl, Phenyl welches ein- bis dreifach durch Fluor, Chlor, Brom, Nitro, Cyano, Hydroxy, Methyl, Ethyl, n-Propyl, i-Propyl, n-Butyl, i-Butyl, s-Butyl, tert.-Butyl, Trifluormethyl, Methoxy, Ethoxy, n-Propoxy, iso-Propoxy, n-Butoxy, iso-Butoxy, sec-Butoxy, tert-Butoxy, Trifluormethoxy, Methylthio, Ethylthio, n-Propylthio, iso-Propylthio, Trifluormethylthio, Formyl, Acetyl, Acetyloxy, Methoxycarbonyl, Ethoxycarbonyl, Carboxy, Amino, Methylamino, Ethylamino, n-Propylamino, iso-Propylamino, Dimethylamino, Diethylamino, Methylethylamino, Di-n-propylamino Di-iso-propylamino substituiert ist,
oder
- R¹: für unsubstituiertes Phenyl oder für Phenyl steht, welches ein- bis dreifach substituiert ist durch Fluor, Chlor, Brom, Nitro, Cyano, Hydroxy, Methyl, Ethyl, n-Propyl, i-Propyl, n-Butyl, i-Butyl, s-Butyl, tert.-Butyl, Trifluormethyl, Methoxy, Ethoxy, n-Propoxy, iso-Propoxy, n-Butoxy, iso-Butoxy, sec-Butoxy, tert-Butoxy, Trifluormethoxy, Methylthio, Ethylthio, n-Propylthio, iso-Propylthio, Trifluormethylthio, Formyl, Acetyl, Acetyloxy, Methoxycarbonyl, Ethoxycarbonyl, Carboxy, Amino, Methylamino, Ethylamino, n-Propylamino, iso-Propylamino, Dimethylamino, Diethylamino, Methylethylamino, Di-n-propylamino Di-iso-propylamino.

Die in den jeweiligen Definitionen bzw. bevorzugten und besonders bevorzugten Definitionen angegebenen Reste können unabhängig von der jeweilig angegebenen Kombination, beliebig auch durch Restedefinitionen anderer Kombinationen ersetzt werden. Außerdem können auch Restedefinitionen aus jedem Vorzugsbereich entfallen.

Die Verbindungen der Formel (I), worin R¹ die oben angegebenen allgemeinen und bevorzugten Bedeutungen hat, sind neu, mit Ausnahme derVerbindungen:
**5-Iod-1-[(4-methoxyphenyl)methyl)-1*H*-tetrazol (CAS164589-78-0)**
**5,5'-Bisiod-1,1'-(ethandiyl)-1*H*-tetrazole(CAS 141651-17-4)**
5-Iod-1-[(phenylmethoxy)methyl]-tetrazol (CAS RN: 208122-86-5)
5-Iod-1-vinyl-tetrazol (CAS RN:141651-20-9)
1-Allyl-5-iod-tetrazol (CAS RN: 141651-19-6)
1-tert-Butyl-5-iod-tetrazol (CAS RN: 141651-18-5)
1-Ethyl-5-iod-tetrazol (CAS RN: 123366-50-7)
5-Iod-tetrazol (CAS RN: 66924-15-0)
1-Methyl-5-iod-tetrazol (CAS RN: 33452-18-5)
5-Iod-1-phenyl-tetrazol (CAS RN: 16484-16-5).

Ein weiterer Gegenstand der vorliegenden Erfindung sind daher die neuen Verbindungen der Formel (I) und Verfahren zu deren Herstellung.

Die Verbindungen der allgemeinen Formel (I) lassen sich herstellen, indem man Tetrazole der allgemeinen Formel (II), 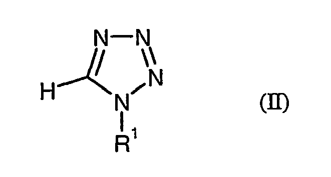 in welcher
- R¹: die oben angegebene allgemeine oder bevorzugte Bedeutung hat,
mit Iod, gegebenenfalls in Gegenwart einer Base oder eines Verdünnungsmittels behandelt.

Im Allgemeinen kann man bei den Verfahren die Temperatur über einen breiten Bereich variieren. In der Regel führt man die Reaktion zwischen 30°C und -100°C durch, bevorzugt bei 0°C bis -90°C, ganz besonders bevorzugt wird in einem Bereich von -20°C und -80°C gearbeitet.

Als Basen können prinzipiell alle gebräuchlichsten Basen eingesetzt werden. Als besonders vorteilhaft haben sich starke Basen erwiesen, wie Alkalimetallamide und Alkalimetallalkylverbindungen. Als ganz besonders bevorzugt seien genannt: Lithiumdüsopropylamid, Methyllithium, Ethyllithium, Propyllithium, n-Butyllithium, tert.-Butyllithium, Natrium, Lithiumhexamethyldisilazid.

Als Verdünnungsmittel können alle Lösungsmittel verwendet werden, die selbst keine Reaktion mit Iod oder der gegebenenfalls eingesetzten Base zeigen. Hierzu gehören vorzugsweise Kohlenwasserstoffe wie Toluol, Xylol oder Hexan, chlorierte Kohlenwasserstoffe wie Chlorbenzol, Methylenchlorid oder Chloroform, Ether wie Tetrahydrofuran, Diethylether, Methyl-tert.-butylether und Dioxan, Nitrile wie Acetonitril, sowie DMSO, DMF und NMP.

Alternativ kann man die Verbindungen der Formel (I) auch erhalten, wenn man Isocyanide der Formel (III)

R¹-NC (III)

in welcher
- R¹: die oben angegebene Bedeutung hat,
mit Aziden und N-Iodsuccinimid, gegebenenfalls in Gegenwart eines Phasentransferkatalysators, umsetzt.

Im Allgemeinen kann man bei diesem Verfahren die Temperatur über einen breiten Bereich variieren, üblicherweise führt man die Reaktion zwischen -20°C und 60°C durch, bevorzugt bei - 10°C bis 40°C.

Als Phasentranferkatalysatoren kann man alle gebräuchlichen Phasentransferkatalysatoren verwenden, wobei Tetraalkylammoniumverbindungen oder Kronenether bevorzugt sind. Als besonders geeignet hat sich Tetrabutylammoniumiodid erwiesen.

Als Lösungsmittel können hierbei alle gebräuchlichen Lösungsmittel verwendet werden, die sich nicht mit Wasser mischen und die nicht selbst mit den eingesetzten Edukten in Reaktion treten. Bevorzugt setzt man chlorierte Kohlenwasserstoffe ein, besonders bevorzugt Chlorbenzol, Methylenchlorid oder Chloroform.

Ferner kann man die Verbindungen der allgemeinen Formel (I) erhalten, wenn man Tetrazole der allgemeinen Formel (II), in welcher R¹ die angegebene Bedeutung hat,
mit Iod gegebenenfalls in Gegenwart einer Säure und gegebenenfalls in Gegenwart eines Oxidationsmittels behandelt.

Im Allgemeinen arbeitet man hierbei zwischen 0°C und 150°C, bevorzugt zwischen 20°C und 130°C und besonders bevorzugt zwischen 80°C und 110°C.

Als Säuren können alle gebräuchlichen Säuren verwendet werden. Bevorzugt setzt man Essigsäure oder Schwefelsäure ein.

Als Oxidationsmittel könne alle gebräuchlichen Oxidationsmittel eingesetzt werden, bevorzugt verwendet man KMnO₄, HNO₃, H₂O₂ oder Peressigsäure, ganz besonders bevorzugt wird KMnO₄ verwendet.

Die Verbindungen der Formel (I) weisen eine starke mikrobizide Wirkung auf und können zur Bekämpfung von unerwünschten Mikroorganisman, wie z.B. Pilzen, Bakterien und Algen eingesetzt werden. Bevorzugt werden die Verbindungen der allgemeinben Formel (I) zur Bekämpfung unerwünschter Mikroorganismen im Materialschutz eingesetzt.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise handelt es sich bei den technischen Materialien um Klebstoffe, Leime, Papier und Karton, Textilien, Leder, Holz, Holzwerkstoffe, Anstrichmittel und Kunststoffartikel, Kühlschmierstoffe und andere Materialien, die von Mikroorganismen befallen oder zersetzt werden können. Weiterhin sind unter technischen Materialien im Rahmen der vorliegenden Erfindung auch Teile von Produktionsanlagen, beispielsweise Kühlwasserkreisläufe, zu verstehen, die durch Vermehrung von Mikroorganismen beeinträchtigt werden können. Bevorzugt zu schützende technische Materialien sind Klebstoffe, Leime, Papiere und Kartone, Leder, Holz, Anstrichmittel, Kunststoffartikel, Kühlschmiermittel und Wärmeübertragungsflüssigkeiten.

Insbesondere eignen sich die erfindungsgemäßen Verbindungen der allgemeinen Formel (I) zum Schutz von Holz, Kunststoffen, Kühlschmiermitteln, wäßrigen organischen oder anorganischen Dispersionen und Beschichtungssystemen wie Anstrichfarben, Lacken oder Putzen vor dem Befall durch Mikroorganismen.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Bakterien.

Es seien beispielsweise Mikroorganismen der folgenden Gattung genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die erfindungsgemäßen Verbindungen (I) können einzeln oder in beliebigem Gemisch untereinander zum Schutz von technischen Materialien eingesetzt werden.

Weiterhin können die erfindungsgemäßen Verbindungen oder deren Mischungen in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole und Feinstverkapselungen in polymeren Stoffen.

Diese Formulierungen können in bekannter Weise hergestellt werden, z.B. durch Vermischen der Einzelwirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0.1 und 95 Gew.-% Wirkstoff oder Wirkstoffmischung, vorzugsweise zwischen 2 und 75 Gew.-%.

Gegenstand der vorliegenden Erfindung sind weiterhin mikrobizide Mittel auf Basis der erfindungsgemäßen Verbindungen, enthaltend mindestens ein Lösungs- oder Verdünnungsmittel sowie gegebenenfalls Verarbeitungshilfsmittel und gegebenenfalls weitere antimikrobiell wirksame Stoffe. Hierbei können die Wirkstoffe hierin entweder in gelöster Form vorliegen oder als Suspensionen oder Emulsionen. Die Lösungs- oder Verdünnungsmittel sind entweder Wasser oder alle gebräuchlichen organischen Lösungsmittel.

Die Wirksamkeit und das Wirkungsspektrum der Wirkstoffe der Formel (I) bzw. die daraus herstellbaren Mittel, Vorprodukte oder ganz allgemein Formulierungen können erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. des zusätzlichen Schutzes vor Insekten zugesetzt werden. Diese Mischungen können ein breiteres Wirkungsspektrum besitzen als die erfindungsgemäßen Verbindungen.

In vielen Fällen erhält man dabei synergistische Effekte, d.h. die Wirksamkeit der Mischung ist größer als die Wirksamkeit der Einzelkomponenten. Besonders günstige Mischungspartner sind z.B. die folgenden Verbindungen:
Triazole wie:
   Azaconazol, Azocyclotin, Bitertanol, Bromuconazol, Cyproconazol, Diclobutrazol, Difenoconazol, Diniconazol, Epoxyconazol, Etaconazol, Fenbuconazol, Fenchlorazol, Fenethanil, Fluquinconazol, Flusilazol, Flutriafol, Furconazol, Hexaconazol, Imibenconazol, Ipconazol, Isozofos, Myclobutanil, Metconazol, Paclobutrazol, Penconazol, Propioconazol, Prothioconazol, Simeoconazol, (±)-cis-1-(4-chlorphenyl)-2-(1H-1,2,4-triazol-1-yl)-cycloheptanol, 2-(1-tert-Butyl)-1-(2-chlorphenyl)-3-(1,2,4-triazol-1-yl)-propan-2-ol, Tebuconazol, Tetraconazol, Triadimefon, Triadimenol, Triapenthenol, Triflumizol, Triticonazol, Uniconazol sowie deren Metallsalze und Säureaddukte;
Imidazole wie:
   Clotrimazol, Bifonazol, Climbazol, Econazol, Fenapamil, Imazalil, Isoconazol, Ketoconazol, Lombazol, Miconazol, Pefurazoat, Prochloraz, Triflumizol, Thiazolcar 1-Imidazolyl-1-(4'-chlorophenoxy)-3,3-dimethylbutan-2-on sowie deren Metallsalze und Säureaddukte;
Pyridine und Pyrimidine wie:
   Ancymidol, Buthiobat, Fenarimol, Mepanipyrin, Nuarimol, Pyroxyfur, Triamirol;
Succinat-Dehydrogenase Inhibitoren wie:
   Benodanil, Carboxim, Carboximsulfoxid, Cyclafluramid, Fenfuram, Flutanil, Furcarbanil, Furmecyclox, Mebenil, Mepronil, Methfuroxam, Metsulfovax, Nicobifen, Pyrocarbolid, Oxycarboxin, Shirlan, Seedvax;
Naphthalin-Derivate wie:
   Terbinafin, Naftifin, Butenaim, 3-Chloro-7-(2-aza-2,7,7-trimethyl-oct-3-en-5-in);
Sulfenamide wie:
   Dichlorfluanid, Tolylfluanid, Folpet, Fluorfolpet; Captan, Captofol;
Benzimidazole wie:
   Carbendazim, Benomyl, Fuberidazole, Thiabendazol oder deren Salze;
Morpholinderivate wie:
   Aldimorph, Dimethomorph, Dodemorph, Falimorph, Fenpropidin, Fenpropimorph, Tridemorph, Trimorphamid und ihre arylsulfonsauren Salze, wie z.B. p-Toluolsulfonsäure und p-Dodecylphenylsulfonsäure;
Benzthiazole wie:
   2-Mercaptobenzothiazol;
Benzthiophendioxide wie:
   Benzo[b]thiophen-S,S-dioxid-carbonsäurecyclohexylamid;
Benzamide wie:
   2,6-Dichloro-N-(4-trifluoromethylbenzyl)-benzamid, Tecloftalam;
Borverbindungen wie:
   Borsäure, Borsäureester, Borax;
Formaldehyd und Formaldehydabspaltende Verbindungen wie:
   Benzylalkoholmono-(poly)-hemiformal, 1,3-Bis(hydroxymethyl)-5,5-dimethylimidazolidine-2,4-dione (DMDMH), Bisoxazolidine, n-Butanol-hemiformal, Cis 1-(3-Chlorallyl)-3,5,7-triaza-1-azoniaadamantane chlorid, 1-[1,3-Bis(hydroxymethyl-2,5-dioxoimidazolidin-4-yl]-1,3-bis(hydroxymethyl)urea, Dazomet, Dimethylolharnstoff, 4,4-Dimethyl-oxazolidine, Ethylenglycol-hemiformal, 7-Ethylbicyclooxazolidine, Hexa-hydro-S-triazine, Hexamethylentetramin, N-Hydroxymethyl-N'-methylthioharnstoff, Methylenbismorpholin, Natrium N-(Hydroxymethyl)glycinat, N-Methylolchloracetamid, Oxazolidine, Paraformaldehyd, Taurolin, Tetrahydro-1,3-oxazin, N-(2-Hydroxypropyl)-amin-methanol, Tetramethylol-acetylen-diharnstoff (TMAD);
Isothiazolinone wie:
   N-Methylisothiazolin-3-on, 5-Chlor-N-methylisothiazolin-3-on, 4,5-Dichloro-N-octylisothiazolin-3-on, 5-Chlor-N-octylisothiazolinon, N-Octyl-isothiazolin-3-on, 4,5-Trimethylen-isothiazolinon, 4,5-Benzisothiazolinon;
Aldehyde wie:
   Zimtaldehyd, Formaldehyd, Glutardialdehyd, β-Bromzimtaldehyd, o-Phthaldialdehyd;
Thiocyanate wie:
   Thiocyanatomethylthiobenzothiazol, Methylenbisthiocyanat;
quartäre Ammoniumverbindungen und Guanidine wie:
   Benzalkoniumchlorid, Benzyldimethyltetradecylammoniumchlorid, Benzyldimethyldodecylammoniumchlorid, Dichlorbenzyldimethylalkylammoniumchlorid, Didecyldimethylammoniumchlorid, Dioctyldimethylammoniumchlorid, N-Hexadecyltrimethylammoniumchlorid, 1-Hexadecylpyridiniumchlorid, Iminoctadine-tris(albesilat);
Iodderivate wie:
   Düodmethyl-p-tolylsulfon, 3-Iod-2-propinyl-alkohol, 4-Chlorphenyl-3-iodpropargylformal, 3-Brom-2,3-diiod-2-propenylethylcarbamat, 2,3,3-Triiodallylalkohol, 3-Brom-2,3-diiod-2-propenylalkohol, 3-Iod-2-propinyl-n-butylcarbamat, 3-Iod-2-propinyl-n-hexylcarbamat, 3-Iod-2-propinyl-cyclohexylcarbamat, 3-Iod-2-propinyl-phenylcarbamat;
Phenole wie:
   Tribromphenol, Tetrachlorphenol, 3-Methyl-4-chlorphenol, 3,5-Dimethyl-4-chlorphenol, Dichlorphen, 2-Benzyl-4-chlorphenol, Triclosan, Diclosan, Hexachlorophen, p-Hydroxybenzoesäuremethylester, p-Hydroxybenzoesäureethylester, p-Hydroxybenzoesäurepropylester, p-Hydroxybenzoesäurebutylester, p-Hydroxybenzoesäureoctylester ,o-Phenylphenol, m-Phenylphenol, p-Phenylphenol, 4-(2-tert.-Butyl-4-methyl-phenoxy)-phenol, 4-(2-Isopropyl-4-methylphenoxy)-phenol, 4-(2,4-Dimethyl-phenoxy)-phenol und deren Alkali- und Erdalkalimetallsalze;
Mikrobizide mit aktivierter Halogengruppe wie:
   Bronopol, Bronidox, 2-Brom-2-nitro-1,3-propandiol, 2-Brom-4'-hydroxy-acetophenon, 1-Brom-3-chlor-4,4,5,5-tetramethyl-2-imidazoldinone, β-Brom-β-nitrostyrol, Chloracetamid, Chloramin T, 1,3-Dibrom-4,4,5,5-tetrametyl-2-imidazoldinone, Dichloramin T, 3,4-Dichlor-(3H)-1,2-dithiol-3-on, 2,2-Dibrom-3-nitril-propionamid, 1,2-Dibrom-2,4-dicyanobutan, Halane, Halazone, Mucochlorsäure, Phenyl-(2-chlor-cyan-vinyl)sulfon, Phenyl-(1,2-dichlor-2-cyanvinyl)sulfon, Trichlorisocyanursäure;
Pyridine wie:
   1-Hydroxy-2-pyridinthion (und ihre Cu-, Na-, Fe-, Mn-, Zn-Salze), Tetrachlor-4-methylsulfonylpyridin, Pyrimethanol, Mepanipyrim, Dipyrithion, 1-Hydroxy-4-methyl-6-(2,4,4-trimethylpentyl)-2(1H)-pyridin;
Methoxyacrylate oder Ähnliche wie:
   Azoxystrobin, Dimoxystrobin, Fluoxastrobin, Kresoxim-methyl, Metominostrobin, Orysastrobin, Picoxystrobin, Pyraclostrobin, Trifloxystrobin, 2,4-dihydro-5-methoxy-2-methyl-4-[2-[[[[1-[3-(trifluoromethyl)phenyl]ethylidene]amino]oxy]methyl]phenyl]-3H-1,2,4-triazol-3-one (CAS-Nr. 185336-79-2);
Metallseifen wie:
   Salze der Metalle Zinn, Kupfer und Zink mit höheren Fett-, Harz-, Naphthensäuren und Phosphorsäure wie z.B. Zinn-, Kupfer-, Zinknaphtenat, -octoat, 2-ethylhexanoat, -oleat, -phosphat, -benzoat;
Metallsalze wie:
   Salze der Metalle Zinn, Kupfer, Zink, sowie auch Chromate und Dichromate wie z.B. Kupferhydroxycarbonat, Natriumdichromat, Kaliumdichromat, Kaliumchromat, Kupfersulfat, Kupferchlorid, Kupferborat, Zinkfluorosilikat, Kupferfluorosilikat;
Oxide wie:
   Oxide der Metalle Zinn, Kupfer und Zink wie z.B. Tributylzinnoxid, Cu₂O, CuO, ZnO;
Oxidationsmittel wie:
   Wasserstoffperoxid, Peressigsäure, Kalium-persulfat;
Dithiocarbamate wie:
   Cufraneb, Ferban, Kalium-N-hydroxymethyl-N'-methyl-dithiobarbamat, Na- oder K-dimethyldithiocarbamat, Macozeb, Maneb, Metam, Metiram, Thiram, Zineb, Ziram;
Nitrile wie:
   2,4,5,6-Tetrachlorisophthalodinitril, Dinatrium-cyano-dithioimidocarbamat;
Chinoline wie:
   8-Hydroxychinolin und deren Cu-Salze;
sonstige Fungizide und Bakterizide wie:
   Bethoxazin, 5-Hydroxy-2(5H)-furanon; 4,5-Benzdithiazolinon, 4,5-Trimethylendithiazolinon, N-(2-p-Chlorbenzoylethyl)-hexaminiumchlorid, 2-Oxo-2-(4-hydroxy-phenyl)acethydroximsäurechlorid, Tris-N-(cyclohexyldiazeniumdioxy)-aluminium, N-(Cyclo-hexyldiazeniumdioxy)-tributylzinn bzw. K-Salze, Bis-N-(cyclohexyldiazeniumdioxy)-kupfer, Iprovalicarb, Fenhexamid, Spiroxamin, Carpropamid, Diflumetorin, Quinoxyfen, Famoxadone, Polyoxorim, Acibenzolar-S-methyl, Furametpyr, Thifluzamide, Methalaxyl-M, Benthiavalicarb, Metrafenon, Cyflufenamid, Tiadinil, Teebaumöl, Phenoxyethanol,
Ag, Zn oder Cu-haltige Zeolithe allein oder eingeschlossen in polymere Werkstoffe.

Ganz besonders bevorzugt sind Mischungen mit
Azaconazol, Bromuconazol, Cyproconazol, Dichlobutrazol, Diniconazol, Diuron, Hexaconazol, Metaconazol, Penconazol, Propiconazol, Tebuconazol, Dichlofluanid, Tolylfluanid, Fluorfolpet, Methfuroxam, Carboxin, Benzo[b]thiophen-S,S-dioxid-carbonsäurecyclohexylamid, Fenpiclonil, 4-(2,2-Difluoro-1,3-benzodioxol-4-yl)-1H-pyrrol-3-carbonitril, Butenafin, Imazalil, N-Methylisothiazolin-3-on, 5-Chlor-N-methylisothiazolin-3-on, N-Octylisothiazolin-3-on, Dichlor-N-octylisozhiazolinon, Mercaptobenzthiazol, Thiocyanatomethylthiobenzothiazol, Thiabendazol, Benzisothiazolinon, N-(2-Hydroxypropyl)-amino-methanol, Benzylalkohol-(hemi)-formal, N-Methylolchloracetamid, N-(2-Hydroxypropyl)-amin-methanol, Glutaraldehyd, Omadine, Zn-Omadine, Dimethyldicarbonat, 2-Brom-2-nitro-1,3-propandiol, 3-Iodo-2-propinyl-n-butylcarbamat, Bethoxazin, o-Phthaldialdehyd, 2,2-Dibrom-3-nitril-propionamid, 1,2-Dibrom-2,4-dicyanobutan, 1,3-Bis(hydroxymethyl)-5,5-dimethylimidazolidine-2,4-dione (DMDMH), Tetramethylol-acetylen-diharnstoff (TMAD), , Ethylenglycol-hemiformal, p-Hydroxybenzoesäure, Carbendazim, Chlorophen, 3-Methyl-4-chlorphenol, o-Phenylphenol.

Desweiteren werden neben den oben genannten Fungiziden und Bakteriziden auch gut wirksame Mischungen mit anderen Wirkstoffen hergestellt:
Insektizide / Akarizide / Nematizide:
   Abamectin, Acephat, Acetamiprid, Acetoprole, Acrinathrin, Alanycarb, Aldicarb, Aldoxycarb, Aldrin, Allethrin, Alpha-cypermethrin, Amidoflumet, Amitraz, Avermectin, Azadirachtin, Azinphos A, Azinphos M, Azocyclotin,
   Bacillus thuringiensis, Barthrin, 4-Bromo-2(4-chlorphenyl)-1-(ethoxymethyl)-5-(trifluoromethyl)-1H-pyrrole-3-carbonitrile, Bendiocarb, Benfuracarb, Bensultap, Betacyfluthrin, Bifenthrin, Bioresmethrin, Bioallethrin, Bistrifluron, Bromophos A, Bromophos M, Bufencarb, Buprofezin, Butathiophos, Butocarboxin, Butoxycarboxim,
   Cadusafos, Carbaryl, Carbofuran, Carbophenothion, Carbosulfan, Cartap, Chinomethionat, Cloethocarb, 4-Chlor-2-(2-chlor-2-methylpropyl)-5-[(6-iod-3-pyridinyl)methoxy]-3(2H)- pyridazinone (CAS-RN: 120955-77-3), Chlordane, Chlorethoxyfos, Chlorfenapyr, Chlorfenvinphos, Chlorfluazuron, Chlormephos, N-[(6-Chloro-3-pyridinyl)-methyl]-N'-cyano-N-methyl-ethanimid-amid, Chlorpicrin, Chlorpyrifos A, Chlorpyrifos M, Cis-Resmethrin, Clocythrin, Clothiazoben, Cypophenothrin Clofentezin, Coumaphos, Cyanophos, Cycloprothrin, Cyfluthrin, Cyhalothrin, Cyhexatin, Cypermethrin, Cyromazin,
   Decamethrin, Deltamethrin, Demeton M, Demeton S, Demeton-S-methyl, Diafenthiuron, Dialiphos, Diazinon, 1,2-Dibenzoyl-1(1,1-dimethyl)-hydrazin, DNOC, Dichlofenthion, Dichlorvos, Dicliphos, Dicrotophos, Difethialon, Diflubenzuron, Dimethoat, 3,5-Dimethylphenylmethylcarbamat, Dimethyl-(phenyl)-silyl-methyl-3-phenoxybenzylether, Dimethyl-(4-Ethoxyphenyl)-silylmethyl-3-phenoxybenzylether, Dimethylvinphos, Dioxathion, Disulfoton,
   Eflusilanate, Emamectin, Empenthrin, Endosulfan, EPN, Esfenvalerat, Ethiofencarb, Ethion, Ethofenprox, Etrimphos, Etoxazole, Etobenzanid,
   Fenamiphos, Fenazaquin, Fenbutatinoxid, Fenfluthrin, Fenitrothion, Fenobucarb, Fenothiocarb, Fenoxycarb, Fenpropathrin, Fenpyrad, Fenpyroximat, Fensulfothion, Fenthion, Fenvalerate, Fipronil, Flonicamid, Fluacrypyrim, Fluazuron, Flucycloxuron, Flucythrinate, Flufenerim, Flufenoxuron, Flupyrazofos, Flufenzine, Flumethrin Flufenprox, Fluvalinate, Fonophos, Formethanate, Formothion, Fosmethilan Fosthiazat, Fubfenprox, Furathiocarb,
   Halofenocid, HCH (CAS RN: 58-89-9), Heptenophos, Hexaflumuron, Hexythiazox, Hydramethylnon, Hydropren,
   Imidacloprid, Imiprothrin, Indoxycarb, Iodfenfos, Iprinomectin, Iprobenfos, Isazophos, Isoamidophos, Isofenphos, Isoprocarb, Isoprothiolane, Isoxathion, Ivermectin,
   Kadedrin
   Lambda-Cyhalothrin, Lufenuron,
   Malathion, Mecarbam, Mervinphos, Mesulfenphos, Metaldehyd, Methacrifos, Methamidophos, Methidathion, Methiocarb, Methomyl, Metalcarb, Milbemectin, Monocrotophos, Moxiectin,
   Naled, NI 125, Nicotin, Nitenpyram, Noviflumuron,
   Omethoat, Oxamyl, Oxydemethon M, Oxydeprofos,
   Parathion A, Parathion M, Penfluron, Permethrin, 2-(4-Phenoxyphenoxy)-ethyl-ethylcarbamat, Phenthoat, Phorat, Phosalon, Phosmet, Phosphamidon, Phoxim, Pirimicarb, Pirimiphos M, Pirimiphos A, Prallethrin, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyridalyl, Pyrimidifen, Pyriproxifen, Pyrithiobac-natrium
   Quinalphos,
   Resmethrin, Rotenon,
   Salithion, Sebufos, Silafluofen, Spinosad, Spirodiclofen, Spiromesifen, Sulfotep, Sulprofos,
   Tau-fluvalinat, Taroils, Tebufenozide, Tebufenpyrad, Tebupirimphos, Teflubenzuron, Tefluthrin, Temephos, Terbam, Terbufos, Tetrachlorvinphos, Tetramethrin, Tetramethacarb, Thiacloprid, Thiafenox, Thiamethoxam, Thiapronil, Thiodicarb, Thiofanox, Thiazophos, Thiocyclam, Thiomethon, Thionazin, Thuringiensin, Tralomethrin, Transfluthrin, Triarathen, Triazophos, Triazamate, Triazuron, Trichlorfon, Triflumuron, Trimethacarb,
   Vamidothion, Xylylcarb, Zetamethrin;
Molluscizide:
   Fentinacetat, Metaldehyd, Methiocarb, Niclosamid;
   Herbizide und Algizide:
   Acetochlor, Acifluorfen, Aclonifen, Acrolein, Alachlor, Alloxydim, Ametryn, Amidosulfuron, Amitrole, Ammonium sulfamate, Anilofos, Asulam, Atrazine, Azafenidin, Aziptrotryn, Azimsulfuron,
   Benazolin, Benfluralin, Benfuresat, Bensulfuron, Bensulfid, Bentazon, Benzofencap, Benzthiazuron, Bifenox, Bispyribac, Bispyribac-Natrium, Borax, Bromacil, Bromobutide, Bromofenoxim, Bromoxynil, Butachlor, Butamifos, Butralin, Butylat, Bialaphos, Benzoyl-prop, Bromobutide, Butroxydim,
   Carbetamid, Carfentrazone-ethyl, Carfenstrol, Chlomethoxyfen, Chloramben, Chlorbromuron, Chlorflurenol, Chloridazon, Chlorimuron, Chlornitrofen, Chloressigsäure, Chloransulam-methyl, Cinidon-ethyl, Chlorotoluron, Chloroxuron, Chlorpropham, Chlorsulfuron, Chlorthal, Chlorthiamid, Cinmethylin, Cinofulsuron, Clefoxydim, Clethodim, Clomazone, Chlomeprop, Clopyralid, Cyanamide, Cyanazine, Cycloat, Cycloxydim, Chloroxynil, Clodinafop-propargyl, Cumyluron, Clometoxyfen, Cyhalofop, Cyhalofop-butyl, Clopyrasuluron, Cyclosulfamuron,
   Diclosulam, Dichlorprop, Dichlorprop-P, Diclofop, Diethatyl, Difenoxuron, Difenzoquat, Diflufenican, Diflufenzopyr, Dimefuron, Dimepiperate, Dimethachlor, Dimethipin, Dinitramine, Dinoseb, Dinoseb Acetate, Dinoterb, Diphenamid, Dipropetryn, Diquat, Dithiopyr, Diduron, DNOC, DSMA, 2,4-D, Daimuron, Dalapon, Dazomet, 2,4-DB, Desmedipham, Desmetryn, Dicamba, Dichlobenil, Dimethamid, Dithiopyr, Dimethametryn,
   Eglinazin, Endothal, EPTC, Esprocarb, Ethalfluralin, Ethidimuron, Ethofumesat, Ethobenzanid, Ethoxyfen, Ethametsulfuron, Ethoxysulfuron,
   Fenoxaprop, Fenoxaprop-P, Fenuron, Flamprop, Flamprop-M, Flazasulfuron, Fluazifop, Fluazifop-P, Fuenachlor, Fluchloralin, Flufenacet Flumeturon, Fluorocglycofen, Fluoronitrofen, Flupropanate, Flurenol, Fluridone, Flurochloridone, Fluroxypyr, Fomesafen, Fosamine, Fosametine, Flamprop-isopropyl, Flamprop-isopropyl-L, Flufenpyr, Flumiclorac-pentyl, Flumipropyn, Flumioxziig Flurtamon, Flumioxzim, Flupyrsulfuron-methyl, Fluthiacet-methyl,
   Glyphosate, Glufosinate-ammonium
   Haloxyfop, Hexazinon,
   Imazamethabenz, Isoproturon, Isoxaben, Isoxapyrifop, Imazapyr, Imazaquin, Imazethapyr, Ioxynil, Isopropalin, Imazosulfuron, Imazomox, Isoxaflutole, Imazapic,
   Ketospiradox,
   Lactofen, Lenacil, Linuron,
   MCPA, MCPA-hydrazid, MCPA-thioethyl, MCPB, Mecoprop, Mecoprop-P, Mefenacet, Mefluidid, Mesosulfuron, Metam, Metamifop, Metamitron, Metazachlor, Methabenzthiazuron, Methazol, Methoroptryne, Methyldymron, Methylisothiocyanat, Metobromuron, Metoxuron, Metribuzin, Metsulfuron, Molinat, Monalid, Monolinuron, MSMA, Metolachlor, Metosulam, Metobenzuron,
   Naproanilid, Napropamid, Naptalam, Neburon, Nicosulfuron, Norflurazon, Natriumchlorat,
   Oxadiazon, Oxyfluorfen, Oxysulfuron, Orbencarb, Oryzalin, Oxadiargyl,
   Propyzamid, Prosulfocarb, Pyrazolate, Pyrazolsulfuron, Pyrazoxyfen, Pyribenzoxim, Pyributicarb, Pyridat, Paraquat, Pebulat, Pendimethalin, Pentachlorophenol, Pentoxazon, Pentanochlor, Petroleumöle, Phenmedipham, Picloram, Piperophos, Pretilachlor, Primisulfuron, Prodiamine, Profoxydim, Prometryn, Propachlor, Propanil, Propaquizafob, Propazine, Propham, Propisochlor, Pyriminobac-methyl, Pelargonsäure, Pyrithiobac, Pyraflufen-ethyl,
   Quinmerac, Quinocloamine, Quizalofop, Quizalofop-P, Quinchlorac,
   Rimsulfuron
   Sethoxydim, Sifuron, Simazine, Simetryn, Sulfosulfuron, Sulfometuron, Sulfentrazone, Sulcotrione, Sulfosate,
   Teeröle, TCA, TCA-Natrium, Tebutam, Tebuthiuron, Terbacil, Terbumeton, Terbutylazine, Terbutryn, Thiazafluoron, Thifensulfuron, Thiobencarb, Thiocarbazil, Tralkoxydim, Triallate, Triasulfuron, Tribenuron, Triclopyr, Tridiphane, Trietazine, Trifluralin, Tycor, Thdiazimin, Thiazopyr, Triflusulfuron,
   Vernolat.

Die Gewichtsverhältnisse der Wirkstoffe in diesen Wirkstoffkombinationen können in relativ großen Bereichen variiert werden.

Vorzugsweise erhalten die Wirkstoffkombinationen den Wirkstoff zu 0,1 bis 99,9 %, insbesondere zu 1 bis 75 %, besonders bevorzugt 5 bis 50 %, wobei der Rest zu 100 % durch einen oder mehrere der obengenannten Mischungspartner ausgefüllt wird.

Die zum Schutz der technischen Materialien verwendeten mikrobiziden Mittel oder Konzentrate enthalten den Wirkstoff bzw. die Wirkstoffkombination in einer Konzentration von 0,01und 95 Gew.-%, insbesondere 0,1 bis 60 Gew.-%.

Die Anwendungskonzentrationen der zu verwendenden Wirkstoffe bzw. der Wirkstoffkombinationen richtet sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im Allgemeinen liegen die Anwendungskonzentrationen im Bereich von 0,001 bis 5 Gew.-%, vorzugsweise von 0,05 bis 2,0 Gew.-%, bezogen auf das zu schützende Material.

Die erfindungsgemäßen Wirkstoffe bzw. Mittel ermöglichen in vorteilhafter Weise, die bisher verfügbaren mikrobiziden Mittel durch effektivere zu ersetzen. Sie zeigen eine gute Stabilität, geringe Verfärbungstendenz und haben in vorteilhafter Weise ein breites Wirkungsspektrum.

Die Wirkstoffe können als solche, in Form ihrer Formulierungen oder den daraus bereiteten Anwendungsformen, wie gebrauchsfertige Lösungen, Emulsionen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.

Die nachfolgenden Beispiele sollen die vorliegende Erfindung erläutern ohne sie in irgend einer Weise einzuschränken.

### Beispiel 1 (Weg A)

### 5-Iod-1-butyltetrazol

In einer Inertgasatmosphäre werden zu einer auf -78°C gekühlten Lösung von 0.03 mol (3.78 g) 1-Butyltetrazol in 40 ml wasserfreiem Tetrahydrofuran 0.032 mol (20 ml) Butyllithium (1.6M in Hexan) tropfenweise so gegeben, daß die Temperatur der Reaktionsmischung -70 C nicht übersteigt. Man rührt 30 Minuten bei dieser Temperatur und tropft anschließend 0.03 mol (7.61 g) Iod in 10 ml wasserfreiem Tetrahydrofuran zu. Die Reaktionsmischung wird nach 30 minütigem Rühren bei dieser Temperatur auf -30°C erwärmt und vorsichtig mit 1 ml Wasser versetzt. Das Lösungsmittel wird unter vermindertem Druck abdestilliert und der Rückstand in Essigsäureethylester/Wasser aufgenommen. Die wäßrige Phase wird mit Essigsäureethylester extrahiert, die vereinigte organische Phase mit Natriumthiosulfatlösung und gesättigter Natriumchloridlösung gewaschen und über Natriumsulfat getrocknet. Der nach dem Abdestillieren des Lösungsmittels unter vermindertem Druck erhaltene Rückstand wird chromatographisch (Elutionsmittel: Chloroform) gereinigt. Es werden 2.2 g (30 %) 5-Iod-1-butyltetrazol
mit einem Schmelzpunkt von 31 - 37 C erhalten.

### Beispiel 2 (Herstellweg B)

### 5-Iod-1-phenyltetrazol

Zu einer auf 0°C gekühlten Mischung von 9.7 mmol (2.18 g) N-Iodsuccinimid in 10 ml Chloroform wird unter gutem Rühren eine Lösung von 9.7 mmol (0.63 g) Natriumazid in 10 ml Wasser und 0.1 g Tetramethylammoniumiodid gegeben. Nach fünfminütigem Rühren wird eine Lösung von 9.7 mmol (1 g) Phenylisocyanid in Chloroform zugetropft und die Reaktionsmischung 45 Minuten bei 0°C gerührt. Man läßt auf Raumtemperatur erwärmen und gibt weitere 10 ml Chloroform zu der Reaktionsmischung. Die Phasen werden getrennt und die organische wird 3mal mit Wasser, 1mal mit gesättigter Natriumthiosulfatlösung, erneut mit Wasser und anschließend mit gesättigter Natriumchloridlösung gewaschen. Nach dem Trocknen über Natriumsulfat wird das Lösungsmittel unter vermindertem Druck abdestilliert und der Rückstand aus Ethanol oder Benzin (bp.: 80...110°C)/Essigsäureethylester umkristallisiert. Es werden 0.2 g (8 %) 5-Iod-1-phenyltetrazol mit einem Schmelzpunkt von 123 - 129°C erhalten.

Analog zu Beispiel 1 und 2 wurden die in Tabelle 1 genannten Verbindungen hergestellt:

**Tabelle 1 (Beispielverbindungen der Formel 1)**

| **Beispiel Nr.** | **R¹** | **Herstellweg** | **Physikalische Daten** |
|---|---|---|---|
| 1 | -n-C₄H₉ | A | Fp.=31-37°C |
| 2 | 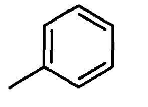 | B | Fp. = 123 -129°C |
| 3 | 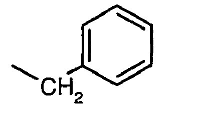 | A | Fp. = 99 -102°C |
| 4 | 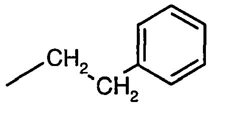 | A | Fp. = 118 - 120°C |
| 5 | 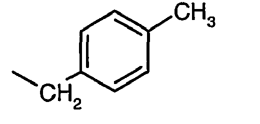 | A | DC: R_{f} = 0,29 (EE/Hexan = 1:1) |
| 6 | 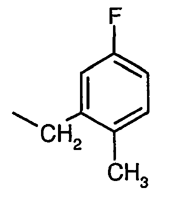 | A | DC: R_{f} = 0,41 (EE/Hexan = 1:2) |
| 7 | 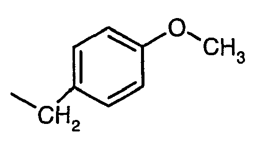 | A | DC: R_{f} = 0,45 (EE/Hexan = 1:1) |
| 8 | 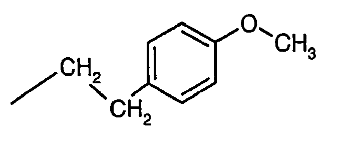 | A | DC: R_{f} = 0,43 (EE/Hexan = 1:1) |
| 9 | 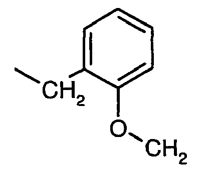 | A | DC: R_{f} = 0,45 (EE/Hexan = 1:1) |
| 10 | 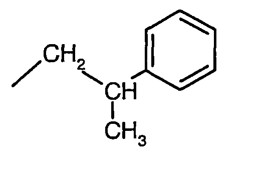 | A | DC: R_{f} = 0,56 (EE/Hexan = 1:1) |
| 11 | 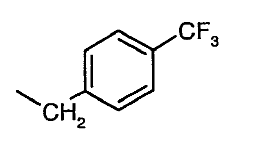 | A | DC: R_{f} = 0,43 (EE/Hexan = 1:1) |
| 12 | 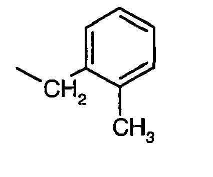 | A | DC: R_{f} = 0,39 (EE/fiexan = 1:2) |
| 13 | 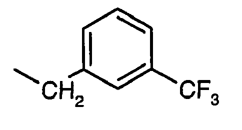 | A | DC: R_{f} = 0,41 (EF/Hexan = 1:2) |
| 14 | 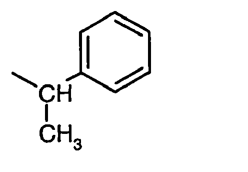 | A | DC: R_{f} = 0,3 (EF/Hexan = 1 : 2) |
| 15 | 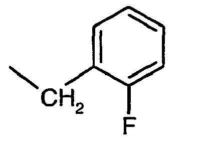 | A | Fp=91°C |
| 16 | 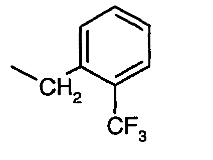 | A | Fp = 103°C |
| 17 | 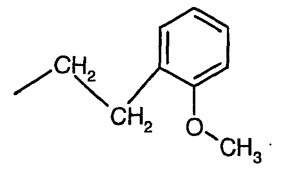 | A | Fp = 80°C |
| 18 | 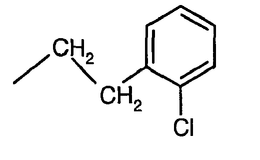 | A | Fp = 96°C |
| 19 | 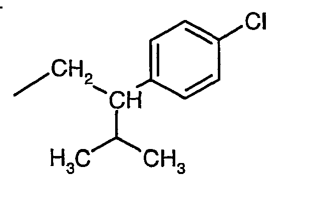 | A | Fp = 111°C |
| 20 | 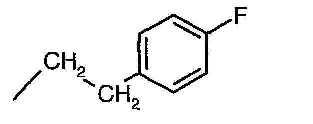 | A | Fp = 137°C |
| 21 | 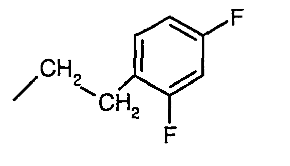 | A | Fp = 107°C |
| 22 | 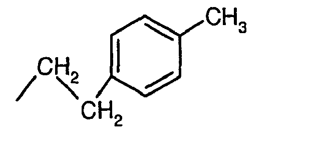 | A | DC: R_{f} = 0,41 (EE/ Hexan) |
| 23 | 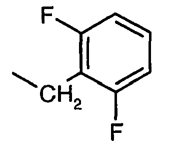 | A | Fp = 102°C |
| 24 | 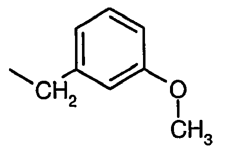 | A | DC: R_{f} = 0,40 (EE/ Hexan) |
| 25 | 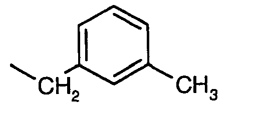 | A | Fp = 85°C |
| 26 | 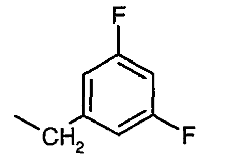 | A | Fp = 135°C |
| 27 | 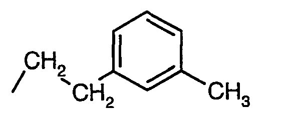 | A | Fp < 50°C |
| 28 | 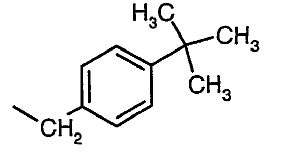 | A | Fp = 120°C |
| 29 | 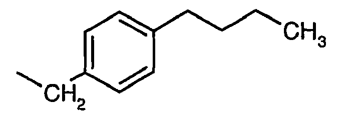 | A | Fp = 79°C |
| 30 | 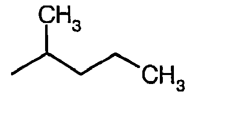 | A | Fp > 260°C |
| 31 | 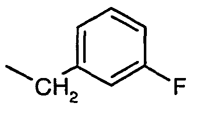 | A | Fp = 45°C |
| 32 | 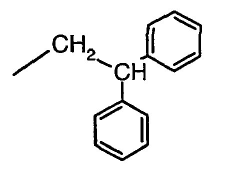 | A | DC: R_{f} = 0,49 (EE/ Hexan) |
| 33 | 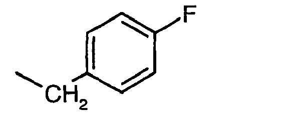 | A | Fp = 109°C |
| 34 | 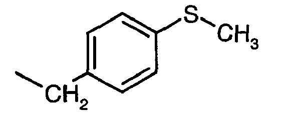 | A | DC: R_{f} = 0,23 (EE/ Hexan) |
| 35 | 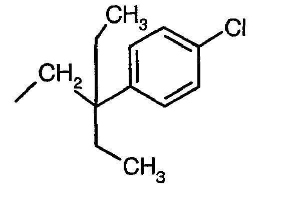 | A | DC :R_{f} = 0,67 (EE/ Hexan) |
| 35 | 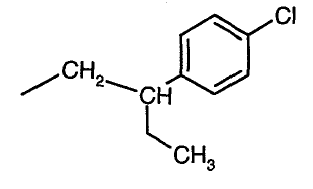 | A | DC: R_{f} = 0,34 (EE/ Hexan) |
| 36 | 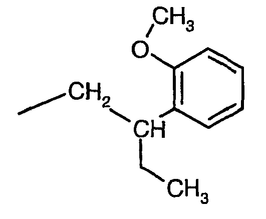 | A | Fp = 123°C |
| 37 | 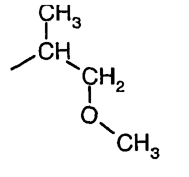 | A | n_{D}= 1,5323 |
| 38 | 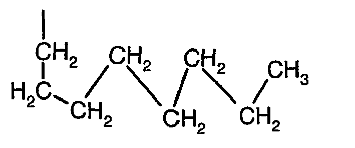 | A | Fp = 40°C |
| 39 | 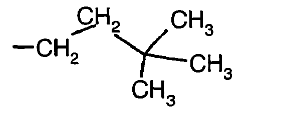 | A | Fp = 125°C |
| 40 | 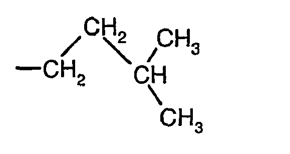 | A | Fp = 63°C |
| 41 | 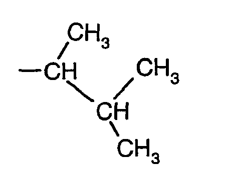 | A | DC: Rf = 0,6 (Tol/EE 5:2) |
| 42 | 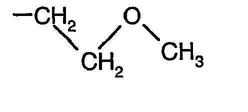 | A | Fp = 94°C |
| 43 | 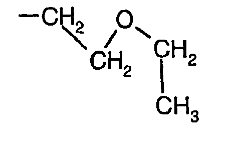 | A | DC: Rf = 0,6 (Tol/EE 5:2) |
| 44 | 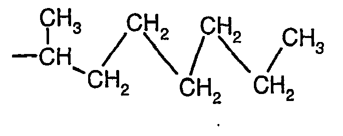 | A | DC: RF = 0,7 (Tol/EE 5:2) |
| 45 | 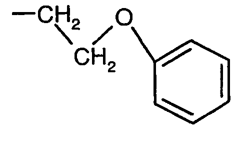 | A | Fp = 106°C |
| 46 | 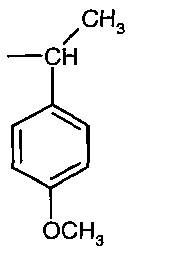 | A | DC: Rf = 0,55 (Tol/EE 5:3) |
| 47 | 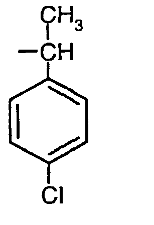 | A | Fp = 77°C |
| 48 | 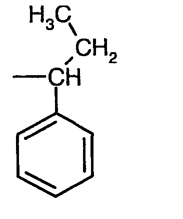 | A | Fp = 67°C |
| 49 | 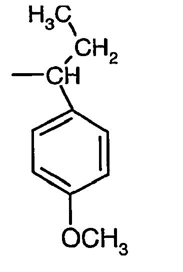 | A | DC: Rf = 0,75 (Tol/EE 5:3) |
| 50 | 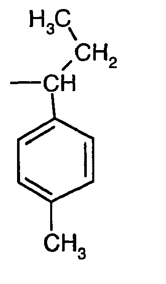 | A | DC: Rf = 0,8 (Tol/EE 5:3) |
| 51 | 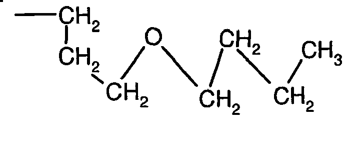 | A | DC= Rf = 0,6 (Tol/EE 5:3) |
| 52 | 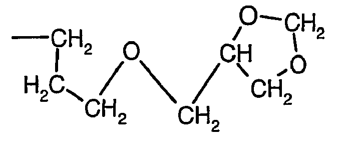 | A | DC: Rf = 0,25 (Tol/EE 5:3) |
| 53 | 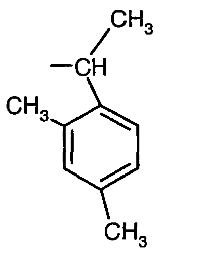 | A | Fp = 100°C |
| 54 | 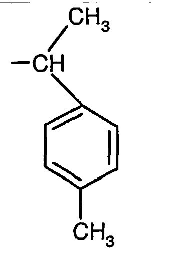 | A | Fp = 95°C |
| 55 | 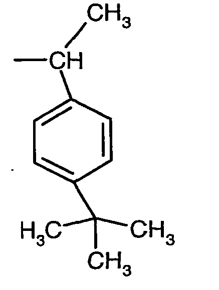 | A | DC: Rf = 0,75 (Tol/EE 5:3) |
| 56 | 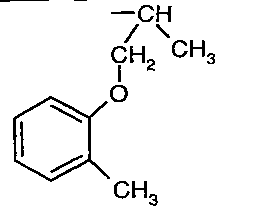 | A | Fp =134°C |
| 57 | 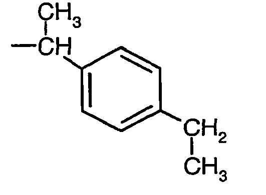 | A | Fp = 50°C |
| 58 | 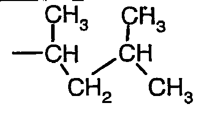 | A | Fp = 60°C |
| 59 | 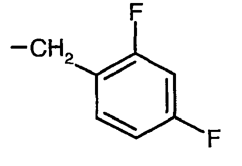 | A | Fp = 88°C |
| 60 | 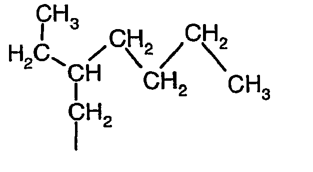 | A | DC: Rf = I |
| 61 | 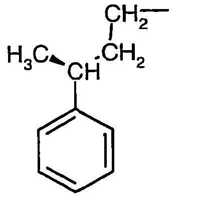 | A | DC: Rf = 0,32 (EE/Hexan = 1:1) |
| 62 | 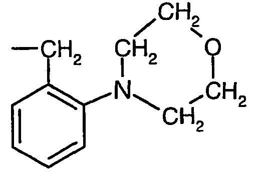 | A | Fp = 148°C |
| 63 | 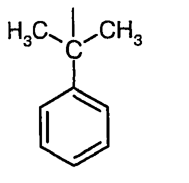 | A | Fp = 130°C |
| 64 | 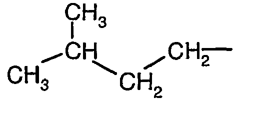 | A | Fp = 58°C |
| 65 | 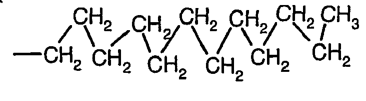 | A | Fp = 52°C |
| 66 | 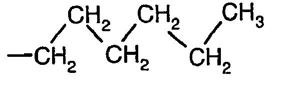 | A | Fp = 32°C |
| 67 | 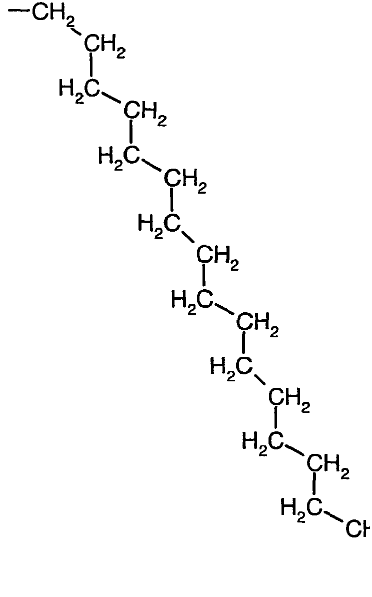 | A | DC: Rf = 0,19 (EE/Hexan = 1:1) |
| 68 | 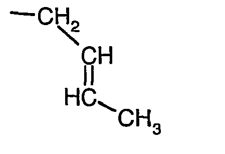 | A | DC: Rf = 0.50 (EE/Hexan 1:1) |

| | | | |
|---|---|---|---|
| DC = Dünnschichtchromatogramm EE = Ethylacetat | | | |

### Anwendungsbeispiel A

Zum Nachweis der Wirksamkeit gegen Pilze wurden die minimalen Hemm-Konzentrationen (MHK) von erfindungsgemäßen Wirkstoffen bestimmt:

Ein Agar, der unter Verwendung von Malzextrakt hergestellt wurde, wurde mit erfindungsgemäßen Wirkstoffen in Konzentrationen von 0,1 mg/l bis 5 000 mg/l versetzt. Nach Erstarren des Agars erfolgte Kontamination mit Reinkulturen der in der Tabelle 3 aufgeführten Testorganismen. Nach 2-wöchiger Lagerung bei 28°C und 60 bis 70 % relativer Luftfeuchtigkeit wurde die MHK bestimmt. Die MHK ist die niedrigste Konzentration an Wirkstoff, bei der keinerlei Bewuchs durch die verwendete Mikrobenart erfolgt. Die ermittelten MHK-Werte sind in der nachstehenden Tabelle 3 angegeben.

**Tabelle 2 Minimale Hemmkonzentrationen (ppm) von erfindungsgemäßen Verbindungen der Formel (I)**

| **Beispiel Nr.** | **Penicillium brevicaule** | **Chaetomium globosum** | **Aspergillus niger** |
|---|---|---|---|
| 1 | <1 | 5 | 5 |
| 2 | <40 | <40 | <40 |
| 3 | 5 | 20 | 20 |
| 4 | 5 | 20 | 20 |
| 5 | <40 | <40 | <40 |

### Anwendungsbeispiel B

Zur Prüfung von Dispersionsanstrichen auf Schimmelfestigkeit wurde wie folgt verfahren:

Das zu prüfende Anstrichmittel wurde beidseitig auf eine geeignete Unterlage gestrichen. Um praxisnahe Ergebnisse zu erhalten, wurde ein Teil der Prüflinge vor dem Test auf Schimmelfestigkeit mit fließendem Wasser (24 h, 20°C) ausgelaugt; ein weiterer Teil wurde mit einem warmen Frischluftstrom behandelt (7 Tage, 40°C).

Die so vorbereiteten Proben wurden daraufhin auf einen Agar-Nährboden gelegt und sowohl Proben als auch Nährboden mit Pilzsporen kontaminiert. Nach 2- 3-wöchiger Lagerung (29 ± 1°C, 80-90 % rel. Luftfeuchte) wurde abgemustert.

Der Anstrich wird dann als dauerhaft schimmelfest eingestuft, wenn die Probe pilzfrei bleibt oder höchstens einen geringen Randbefall erkennen lässt.

Zur Kontamination wurden Pilzsporen folgender Schimmelpilze verwendet, die als Anstrichzerstörer bekannt sind oder häufig auf Anstrichen angetroffen werden:
Alternaria tenuis
Aspergillus flavus
Aspergillus niger
Aspergillus ustus
Cindosporum herbarum
Paecilomyces variotii
Penicillium citrium
Aureobasidium pullulans
Stachybotrys chartarum
Schimmelfest sind Anstriche gemäß Rezeptur A (auch nach Auslaugung und Windkanalexposition) wenn sie beispielsweise 1,0 % (bezogen auf Feststoff) der Beispielverbindungen 2, 3, 4 oder 5 enthalten.

**Rezeptur A: Außendispersionsfarbe auf Basis von Acroal 290 D (Styrolacrylat)**

| **Handelsname** | **Gew.-Teile** | **Chemische Bezeichnung** |
|---|---|---|
| Bayer Titan RKB2 | 40 | Titandioxid |
| Talkum V58 neu | 10 | Magnesiumsilikat wasserhaltig |
| Durcal 5 | 45 | Calcit CaCO₃ |
| Walsroder MC 3000 S 2 %ig | 30 | Methylcellulose |
| H₂O | 6,5 | Destilliertes Wasser |
| Calgon N 10 %ig | 3 | Polyphosphat |
| Pigmentverteiler A 10 %ig | 1 | Polyacrylsäuresalz |
| Agitan 281, 1:1 in Texanol | 1 | |
| Testbenzin | 5 | Gemisch aliph. Kohlenwasserstoffe |
| Butylglykolacetat | 1.5 | Butylglykolacetat |
| Acronal 290 D (Bindemittel) | 71 | Polyacrylsäureester |
| **Gesamt** | **219** | |

| | | |
|---|---|---|
| Feststoffgehalt 135,5 = 61,6%. | | |

## Patentansprüche

1. Verwendung von Verbindungen der Formel (I) 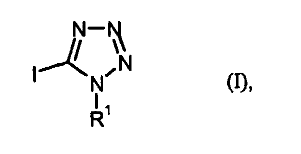 in welcher
R¹ für Wasserstoff oder jeweils gegebenenfalls substituiertes Alkyl, Alkenyl, Alkinyl oder Phenyl steht,
als Mikrobizide zum Schutz von Pflanzen und Materialien.

2. Verwendung gemäß Anspruch 1, **dadurch gekennzeichnet, dass** Verbindungen der Formel (I) gemäß Anspruch 1 eingesetzt werden,
worin
R¹ für Wasserstoff, für geradkettiges oder verzweigtes, unsubstituiertes oder ein- oder mehrfach gleich oder verschieden substituiertes C₁-C₈-Alkyl, für geradkettiges oder verzweigtes, unsubstituiertes oder ein- oder mehrfach gleich oder verschieden substituiertes C₂-C₈-Alkenyl oder für geradkettiges oder verzweigtes, unsubstituiertes oder ein- oder mehrfach gleich oder verschieden substituiertes C₂-C₈-Alkinyl steht,
wobei die Substituenten für die ein- oder mehrfach gleich oder verschieden substituierten Alkyl-, Alkenyl- und Alkinylreste ausgewählt sind aus der Reihe Halogen; Nitro; Cyano; Hydroxy; unsubstituiertes C₁-C₆-Alkoxy; C₁-C₆-Alkoxy welches 1- bis 9-fach, gleich oder verschieden durch Halogen substituiert ist; unsubstituiertes C₁-C₆-Alkylthio; C₁-C₆-Alkylthio welches 1- bis 9-fach, gleich oder verschiedenen durch Halogen substituiert ist; Amino; Monoalkylamino mit geradkettigen oder verzweigten C₁-C₆-Alkylresten; Dialkylamino mit gleichen oder verschiedenen, geradkettigen oder verzweigten C₁-C₆-Alkylresten; unsubstituiertes Phenyl; Phenyl, welches ein bis 5-fach, gleich oder verschieden substituiert ist durch Halogen, Nitro, Cyano, Alkyl, Halogenalkyl, Alkoxy, Halogenalkoxy, Alkylthio, Halogenalkylthio, Acyl, Acyloxy, Alkoxy-carbonyl, Carboxyl, Amino, Monoalkylamino oder Dialkylamino;
oder
R¹ für unsubstituiertes Phenyl oder für Phenyl steht, welches ein bis 5- fach, gleich oder verschieden substituiert ist durch Halogen, Nitro, Cyano, Hydroxy, Alkyl, Halogenalkyl, Alkoxy, Halogenalkoxy, Alkylthio, Halogenalkylthio, Acyl, Acyloxy, Alkoxycarbonyl, Carboxyl, Amino, Monoalkylamino oder Dialkylamino.

3. Verwendung gemäß wenigstens einem der Ansprüche 1 und 2, **dadurch gekennzeichnet, dass** technische Materialien gegen Befall und/oder Zerstörung durch Mikroorganismen geschützt werden.

4. Verwendung gemäß wenigstens einem der Ansprüche 1 bis 3, **dadurch gekennzeichnet, dass** es sich bei den technischen Materialien um Klebstoffe, Leime, Papiere und Kartone, Leder, Holz, Anstrichmittel, Kunststoffartikel, Kühlschmiermittel und Wärmeübertragungsflüssigkeiten handelt

5. Mikrobizides Mittel enthaltend mindestens eine Verbindung der Formel (I) gemäß Anspruch 1 und mindestens ein Lösungs- oder Verdünnungsmittel sowie gegebenenfalls Verarbeitungshilfsmittel und gegebenenfalls weitere antimikrobiell wirksame Stoffe, wobei solche Mikrobizide Mittel ausgenommen sind, die mindestens ein Lösungs- oder Verdünnungsmittel und eine der nachstehenden Verbindungen enthält:
5-Iod-1-[(4-methoxyphenyl)methyl]-1*H*-tetrazol (CAS164589-78-0)
5,5'-Bisiod-1,1'-(ethandiyl)-1*H*-tetrazol (CAS 141651-17-4)
5-Iod-1-[(phenylmethoxy)methyl]-tetrazol (CAS RN: 208122-86-5)
5-Iod-1-vinyl-tetrazol (CAS RN:141651-20-9)
1-Allyl-5-iod-tetrazol (CAS RN: 141651-19-6)
1-tert-Butyl-5-iod-tetrazol (CAS RN: 141651-18-5)
1-Ethyl-5-iod-tetrazol (CAS RN: 123366-50-7)
5-Iod-tetrazol (CAS RN: 66924-15-0)
1-Methyl-5-iod-tetrazol (CAS RN: 33452-18-5)
5-Iod-1-phenyl-tetrazol (CAS RN: 16484-16-5).

6. Verfahren zur Herstellung eines mikrobiziden Mittels gemäß Anspruch 5, **dadurch gekennzeichnet, dass** man mindestens eine Verbindung der Formel (I) gemäß Anspruch 1 und mindestens ein Lösungs- oder Verdünnungsmittel sowie gegebenenfalls Verarbeitungshilfsmittel und gegebenenfalls weitere antimikrobiell wirksame Stoffe miteinander vermischt.

7. Verfahren zum Schutz von Pflanzen und Materialien vor Befall und/oder Zerstörung durch Mikroorganismen, **dadurch gekennzeichnet, dass** man mindestens eine Verbindung der Formel (I) gemäß Anspruch 1 oder ein mikrobizides Mittel gemäß Anspruch 5 auf den Mikoorganismus oder dessen Lebensraum einwirken läßt.

8. Verbindungen der Formel (I) 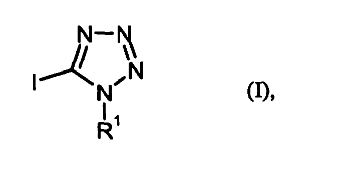 in welcher
R¹ für Wasserstoff oder jeweils gegebenenfalls substituiertes Alkyl, Alkenyl, Alkinyl oder Phenyl steht,
mit Ausnahme der Verbindungen
5-Iod-1-[(4-methoxyphenyl)methyl]-1*H*-tetrazol (CAS164589-78-0)
5,5'-Bisiod-1,1'-(ethandiyl)-1*H*-tetrazol (CAS 141651-17-4)
5-Iod-1-[(phenylmethoxy)methyl]-tetrazol (CAS RN: 208122-8b-5)
5-Iod-1-vinyl-tetrazol (CAS RN:141651-20-9)
1-Allyl-5-iod-tetrazol (CAS RN: 141651-19-6)
1-tert-Butyl-5-iod-tetrazol (CAS RN: 141651-18-5)
1-Ethyl-5-iod-tetrazol (CAS RN: 123366-50-7)
5-Iod-tetrazol (CAS RN: 66924-15-0)
1-Methyl-5-iod-tetrazol (CAS RN: 33452-18-5)
5-Iod-1-phenyl-tetrazol (CAS RN: 16484-16-5).

9. Verfahren zur Herstellung von Verbindungen gemäß Anspruch 8, **dadurch gekennzeichnet, dass** man Tetrazole der allgemeinen Formel (II), 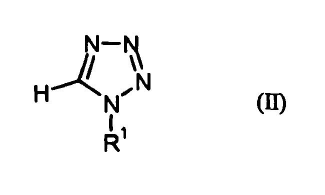 in welcher
R¹ die in Anspruch 1 angegebene Bedeutung hat,
mit Iod, gegebenenfalls in Gegenwart einer Base oder eines Verdünnungsmittels behandelt.

10. Technische Materialien enthaltend wenigstens eine Verbindung gemäß Anspruch 8.

## Claims

1. Use of a compound of the formula (I) 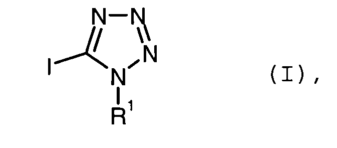 in which
R¹ represents hydrogen or in each case optionally substituted alkyl, alkenyl, alkynyl or phenyl,
as a microbicide for protecting plants and materials.

2. Use according to Claim 1, **characterized in that** a compound of the formula (I) according to Claim 1 is used
in which
R¹ represents hydrogen, represents straight-chain or branched C₁-C₈-alkyl which is unsubstituted or mono- or polysubstituted by identical or different substituents, represents straight-chain or branched C₂-C₈-alkenyl which is unsubstituted or mono- or polysubstituted by identical or different substituents or represents straight-chain or branched C₂-C₈-alkynyl which is unsubstituted or mono- or polysubstituted by identical or different substituents,
where the substituents for the alkyl, alkenyl and alkynyl radicals which are mono- or polysubstituted by identical or different substituents are selected from the group consisting of halogen; nitro; cyano; hydroxyl; unsubstituted C₁-C₆-alkoxy; C₁-C₆-alkoxy which is mono- to nonasubstituted by identical or different halogen substituents; unsubstituted C₁-C₆-alkylthio; C₁-C₆-alkylthio which is mono- to nonasubstituted by identical or different halogen substituents; amino; monoalkylamino having straight-chain or branched C₁-C₆-alkyl radicals; dialkylamino having identical or different straight-chain or branched C₁-C₆-alkyl radicals; unsubstituted phenyl; phenyl which is mono- to pentasubstituted by identical or different substituents from the group consisting of halogen, nitro, cyano, alkyl, haloalkyl, alkoxy, haloalkoxy, alkylthio, haloalkylthio, acyl, acyloxy, alkoxycarbonyl, carboxyl, amino, monoalkylamino and dialkylamino;
or
R¹ represents unsubstituted phenyl or represents phenyl which is mono- to pentasubstituted by identical or different substituents from the group consisting of halogen, nitro, cyano, hydroxyl, alkyl, haloalkyl, alkoxy, haloalkoxy, alkylthio, haloalkylthio, acyl, acyloxy, alkoxycarbonyl, carboxyl, amino, monoalkylamino and dialkylamino.

3. Use according to at least one of Claims 1 and 2, **characterized in that** industrial materials are protected against attack and/or destruction by microorganisms.

4. Use according to at least one of Claims 1 to 3, **characterized in that** the industrial materials are adhesives, sizes, paper and cardboard, leather, wood, paints, plastics, cooling lubricants and heat-transfer liquids.

5. Microbicidal composition, comprising at least one compound of the formula (I) according to Claim 1 and at least one solvent or diluent and also, if appropriate, processing auxiliaries and, if appropriate, further antimicrobially active compounds, in which those microbicidal compositions are excepted which contain at least one solvent or diluent and one of the following compounds:
5-iodo-1-[(4-methoxyphenyl)methyl]-1*H-*tetrazole(CAS164589-78-0)
5,5'-bisiodo-1,1'-(ethanediyl)-1*H*-tetrazole(CAS 141651-17-4)
5-iodo-1-[(phenylmethoxy)methyl]tetrazole (CAS RN: 208122-86-5)
5-iodo-1-vinyltetrazole (CAS RN:141651-20-9)
1-allyl-5-iodotetrazole (CAS RN: 141651-19-6)
1-tert-butyl-5-iodotetrazole (CAS RN: 141651-18-5)
1-ethyl-5-iodotetrazole (CAS RN: 123366-50-7)
5-iodotetrazole (CAS RN: 66924-15-0)
1-methyl-5-iodotetrazole (CAS RN: 33452-18-5)
5-iodo-1-phenyltetrazole (CAS RN: 16484-16-5).

6. Process for preparing a microbicidal composition according to Claim 5, **characterized in that** at least one compound of the formula (I) according to Claim 1 and at least one solvent or diluent and also, if appropriate, processing auxiliaries and, if appropriate, further antimicrobially active compounds are mixed with one another.

7. Method for protecting plants and materials against attack and/or destruction by microorganisms, **characterized in that** at least one compound of the formula (I) according to Claim 1 or a microbicidal composition according to Claim 5 are allowed to act on the microorganism or its habitat.

8. Compound of the formula (I) 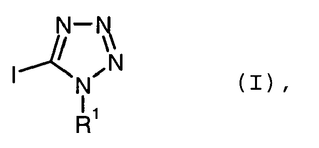 in which
R¹ represents hydrogen or in each case optionally substituted alkyl, alkenyl, alkynyl or phenyl,
except for the compounds
5-iodo-1-[(4-methoxyphenyl)methyl]-1*H-*tetrazole(CAS164589-78-0)
5,5'-bisiodo-1,1'-(ethanediyl)-1*H*-tetrazole(CAS 141651-17-4)
5-iodo-1-[(phenylmethoxy)methyl]tetrazole (CAS RN: 208122-86-5)
5-iodo-1-vinyltetrazole (CAS RN:141651-20-9)
1-allyl-5-iodotetrazole (CAS RN: 141651-19-6)
1-tert-butyl-5-iodotetrazole (CAS RN: 141651-18-5)
1-ethyl-5-iodotetrazole (CAS RN: 123366-50-7)
5-iodotetrazole (CAS RN: 66924-15-0)
1-methyl-5-iodotetrazole (CAS RN: 33452-18-5)
5-iodo-1-phenyltetrazole (CAS RN: 16484-16-5).

9. Process for preparing a compound according to Claim 8, **characterized in that** a tetrazole of the general formula (II) 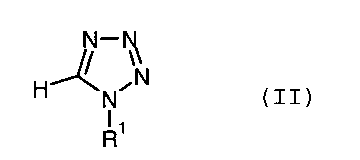 in which
R¹ is as defined in Claim 1
is treated with iodine, if appropriate in the presence of a base or a diluent.

10. Industrial material, comprising at least one compound according to Claim 8.

## Revendications

1. Utilisation de composés de formule (I) 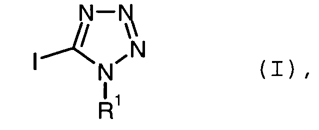 dans laquelle
R¹ représente un atome d'hydrogène ou un groupe alkyle, un groupe alcényle, un groupe alcynyle ou un groupe phényle, dans chaque cas éventuellement substitué,
en tant qu'agents microbicides pour la protection de plantes et de matériaux.

2. Utilisation selon la revendication 1, **caractérisée en ce que** l'on utilise des composés de formule (I) selon la revendication 1,
dans laquelle
R¹ représente un atome d'hydrogène, un groupe alkyle en C₁-C₈ linéaire ou ramifié, non substitué ou mono- ou polysubstitué de façon identique ou différente, un groupe alcényle en C₂-C₈ linéaire ou ramifié, non substitué ou mono- ou polysubstitué de façon identique ou différente, ou un groupe alcynyle en C₂-C₈ linéaire ou ramifié, non substitué ou mono-ou polysubstitué de façon identique ou différente,
où les substituants pour les radicaux alkyle, alcényle et alcynyle mono- ou polysubstitués de façon identique ou différente sont choisis parmi le groupe constitué d'un atome d'halogène ; d'un groupe nitro ; d'un groupe cyano ; d'un groupe hydroxy ; d'un groupe alcoxy en C₁-C₆ non substitué ; d'un groupe alcoxy en C₁-C₆ qui est substitué de 1 à 9 fois, de façon identique ou différente, par un atome d'halogène ; d'un groupe alkylthio en C₁-C₆ non substitué ; d'un groupe alkylthio en C₁-C₆ qui est substitué de 1 à 9 fois, de façon identique ou différente, par un atome d'halogène ; d'un groupe amino ; d'un groupe monoalkylamino renfermant des radicaux alkyle en C₁-C₆ linéaires ou ramifiés ; d'un groupe dialkylamino renfermant des radicaux alkyle en C₁-C₆ identiques ou différents, linéaires ou ramifiés ; d'un groupe phényle non substitué ; d'un groupe phényle qui est substitué jusqu'à 5 fois, de façon identique ou différente, par un atome d'halogène, un groupe nitro, un groupe cyano, un groupe alkyle, un groupe halogénoalkyle, un groupe alcoxy, un groupe halogénoalcoxy, un groupe alkylthio, un groupe halogénoalkylthio, un groupe acyle, un groupe acyloxy, un groupe alcoxycarbonyle, un groupe carboxyle, un groupe amino, un groupe monoalkylamino ou un groupe dialkylamino ;
ou
R¹ représente un groupe phényle non substitué ou un groupe phényle qui est substitué jusqu'à 5 fois, de façon identique ou différente, par un atome d'halogène, un groupe nitro, un groupe cyano, un groupe hydroxy, un groupe alkyle, un groupe halogénoalkyle, un groupe alcoxy, un groupe halogénoalcoxy, un groupe alkylthio, un groupe halogénoalkylthio, un groupe acyle, un groupe acyloxy, un groupe alcoxycarbonyle, un groupe carboxyle, un groupe amino, un groupe monoalkylamino ou un groupe dialkylamino.

3. Utilisation selon au moins l'une des revendications 1 et 2, **caractérisée en ce que** des matériaux industriels sont protégés contre une attaque et/ou une destruction par des micro-organismes.

4. Utilisation selon au moins l'une des revendications 1 à 3, **caractérisée en ce que** les matériaux industriels sont des adhésifs, des colles, des papiers et cartons, du cuir, du bois, des peintures, des articles en plastique, des lubrifiants réfrigérants et des caloporteurs.

5. Composition microbicide contenant au moins un composé de formule (I) selon la revendication 1 et au moins un solvant ou un diluant ainsi qu'éventuellement des auxiliaires de traitement et éventuellement des substances à action antimicrobienne supplémentaires, à l'exception des compositions microbicides qui contiennent au moins un solvant ou un diluant et l'un des composés suivants :
le 5-iodo-1-[(4-méthoxyphényl)méthyl]-1*H*-tétrazole (CAS 164589-78-0)
le 5,5'-bis-iodo-1,1'-(éthanediyl)-1H-tétrazole (CAS 141651-17-4)
le 5-iodo-1-[(phénylméthoxy)méthyl]tétrazole (CAS RN : 208122-86-5)
le 5-iodo-1-vinyltétrazole (CAS RN : 141651-20-9)
le 1-allyl-5-iodotétrazole (CAS RN : 141651-19-6)
le 1-tert-butyl-5-iodotétrazole (CAS RN : 141651-18-5)
le 1-éthyl-5-iodotétrazole (CAS RN : 123366-50-7)
le 5-iodotétrazole (CAS RN : 66924-15-0)
le 1-méthyl-5-iodotétrazole (CAS RN : 33452-18-5)
le 5-iodo-1-phényltétrazole (CAS RN : 16484-16-5).

6. Procédé de préparation d'une composition microbicide selon la revendication 5, **caractérisé en ce qu'**au moins un composé de formule (I) selon la revendication 1 et au moins un solvant ou un diluant ainsi qu'éventuellement des auxiliaires de traitement et éventuellement des substances à action antimicrobienne supplémentaires sont mélangés les uns avec les autres.

7. Procédé de protection de plantes et de matériaux contre une attaque et/ou une destruction par des micro-organismes, **caractérisé en ce que** l'on laisse au moins un composé de formule (I) selon la revendication 1 ou une composition microbicide selon la revendication 5 agir sur le micro-organisme ou son milieu.

8. Composés de formule (I) 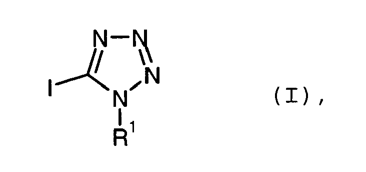 dans laquelle
R¹ représente un atome d'hydrogène ou un groupe alkyle, un groupe alcényle, un groupe alcynyle ou un groupe phényle, dans chaque cas éventuellement substitués,
à l'exception des composés
5-iodo-1-[(4-méthoxyphényl)méthyl]-1*H*-tétrazole (CAS 164589-78-0)
5,5'-bis-iodo-1,1'-(éthanediyl)-1*H*-tétrazole (CAS 141651-17-4)
5-iodo-1-[(phénylméthoxy)méthyl]tétrazole (CAS RN : 208122-86-5)
5-iodo-1-vinyltétrazole (CAS RN : 141651-20-9)
1-allyl-5-iodotétrazole (CAS RN : 141651-19-6)
1-tert-butyl-5-iodotétrazole (CAS RN : 141651-18-5)
1-éthyl-5-iodotétrazole (CAS RN : 123366-50-7)
5-iodotétrazole (CAS RN : 66924-15-0)
1-méthyl-5-iodotétrazole (CAS RN : 33452-18-5)
5-iodo-1-phényltétrazole (CAS RN : 16484-16-5).

9. Procédé de préparation de composés selon la revendication 8, **caractérisé en ce que** des tétrazoles de formule générale (II), 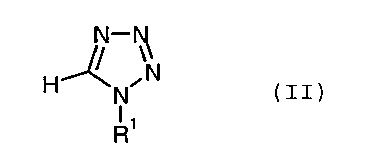 dans laquelle
R¹ présente la signification indiquée dans la revendication 1,
sont traités avec de l'iode, de préférence en présence d'une base ou d'un diluant.

10. Matériaux industriels contenant au moins un composé selon la revendication 8.
